# EUROPEAN PATENT SPECIFICATION

(11) **EP 1 152 741 B1**
(45) Date of publication and mention of the grant of the patent: **25.10.2006**
(21) Application number: 00984412.7
(22) Date of filing: 13.12.2000
(51) Int. Cl.: A61K 8/34, A61K 8/27, A61K 8/92, A61Q 17/00, A61Q 19/00

(54) **STABILIZED ANTIMICROBIAL SYSTEMS AND METHODS OF MAKING THE SAME**
STABILISIERTE ANTIMIKROBIELLE SYSTEME UND VERFAHREN ZUR HERSTELLUNG
SYSTEMES ANTIMICROBIENS STABILISES ET PROCEDES DE PRODUCTION

(30) Priority: 13.12.1999 US 460012
(43) Date of publication of application: 14.11.2001
(73) Proprietor: ETHICON, INC., Somerville, NJ 08876 (US)
(72) Inventor: JAMPANI, Hanuman, Bridgewater, NJ 08807 (US); HOLLY, Thomas, F., Arlington, TX 76014 (US); NEWMAN, Jerry, L., Arlington, TX 76016 (US)
(74) Representative: Mercer, Christopher Paul
(86) International application number: PCT/US2000/034008
(87) International publication number: WO 2001/041727

(56) References cited:
- EP-A- 0 640 285
- EP-A- 0 769 291
- WO-A-97/00667
- WO-A-99/59540

## Description

### BACKGROUND OF THE INVENTION

### 1. Field of the Invention

This invention is concerned with stabilized antimicrobial systems and methods of making the same.

### 2. Related Art

The physical appearance of antimicrobial products, such as hand gels and lotions, is an important consideration of a potential user of the products. While alcohol and alcohol-containing mixtures are known to possess bactericidal activity, such compositions are not as widely accepted for use as non-alcohol antimicrobial products because alcohol and alcohol-containing mixtures dehydrate the skin. The dehydration is caused by the denaturing and delipidizing of the skin's lipid molecules. It is appreciated in the art that lipids in the stratum corneum of the skin are important for the barrier properties of the skin.

A way of overcoming the dehydrating nature of alcohol-containing antimicrobial compositions is described in US-A-5,997,893, entitled ALCOHOL BASED ANTIMICORBIAL COMPOSITIONS WITH COSMETIC APPEARANCE. These alcohol-containing compositions of the foregoing patent are effective as antimicrobial compositions that desirably possess the appearance, feel and moisturizing attributes of a hand cream and lotion. Thus, the appearance of such compositions is an important factor in overcoming the perceived drying attribute of alcohol-containing formulations. Another desirable aspect for the appearance of these formulations is that the appearance be stable, that is not change over time or separate into various phases. It is relatively easy to achieve white, lotion appearance with oil in water and/or water in oil type of emulsion based formulations by adding inorganic fillers or by using compounds that emulsify and offer that look to the end product. However, it is quite complex to obtain such an appearance with an excellent stability profile in a high alcohol system (i.e., 50-90 v/v% active alcohol) having 5 - 15 wt.% water, skin conditioners such as hydrophilic oil (e.g., isolene), fatty acid esters including phosphate esters such as naturally derived synthetic phospholipids (e.g., Phospholipid), humectant (e.g., glycerin), and percutaneous enhancers (propylene glycol). Some of the prototypes prepared using this combination were evaluated for the stability at elevated temperatures (40 degrees), and found to be unstable. Microscopic examination of some of these prototypes has shown separation of isolene in large quantities as an oil at the bottom of the glass vials. According to the present invention there is provided a stable high alcohol-containing antimicrobial composition as defined in the appendant claims.

### BRIEF DESCRIPTION OF THE DRAWINGS

Fig. 1(a) depicts a microscopic view at 200X magnification of unaged Formulation 1-1.
Fig. 1(b) depicts a microscopic view at 200X magnification of Formulation 1-1 after accelerated aging at 40 C for 5 weeks.
Fig. 1(c) depicts a microscopic view at 200X magnification of unaged Formulation 1-2.
Fig. 1(d) depicts a microscopic view at 200X magnification of Formulation 1-2 after accelerated aging at 50 C for 20 weeks.
Fig. 1(e) depicts a microscopic view at 200X magnification of unaged Formulation 1-3.
Fig. 1(f) depicts a microscopic view at 200X magnification of Formulation 1-3 after accelerated aging at 50 C for 20 weeks.
Fig. 2(a) depicts a microscopic view at 100X magnification of Formulation 2-1 after forming a premixed paste.
Fig. 2(b) depicts a microscopic view at 100X magnification of unaged Formulation 2-1 after incorporating the premixed paste and forming a final antimicrobial formulation.
Fig. 3 (a) depicts a microscopic view at 200X magnification of unaged Formulation 3-1.
Fig. 3(b) depicts a microscopic view at 200X magnification of Formulation 3-1 after accelerated aging at 50 C for 3 weeks.
Fig. 3(c) depicts a microscopic view at 200X magnification of unaged Formulation 3-2.
Fig. 3(d) depicts a microscopic view at 200X magnification of Formulation 3-2 after accelerated aging at 50 C for 3 weeks.

### SUMMARY OF THE INVENTION

One aspect of the present invention is based on the surprising discovery that the mere addition of a small amount of a metal oxide in a high alcohol-containing antimicrobial composition comprising at least about 50 to about 90 v/v% (approx. 40 to 70 wt./wt.% based on ethanol) of active alcohol, an effective amount of a hydrophilic oil, an effective amount of a cationic antimicrobial resulted in a stable antimicrobial composition with greatly enhanced product stability in terms of appearance of alcohol-containing antimicrobial compositions. The metal oxides are preferably microfine (i.e., > than about 5 m²/g surface area (S.A.))titanium dioxide and zinc oxide.

### DETAILED DESCRIPTION OF PREFERRED EMBODIMENTS OF THE INVENTION

In one embodiment of the invention, compositions contain an amount of a metal oxide effective to provide physical stability to the composition. Typically the effective amount of metal oxides is from 0.01 to 1.0, preferably from 0.05 to 0.5, and most preferably from 0.1 to 0.25 weight percent of metal oxide based on individual metal oxide.

Suitable metal oxides for use with this invention include transition and post-transition metal oxides. Most preferably the metal oxides are selected from the group consisting of zinc oxide and titanium dioxide. In the case of zinc oxide, a known skin protectant at concentrations of about 1 weight %, zinc oxide is especially preferred because of its natural, mild antibacterial activity with astringent and substantive skin treating properties (i.e., will bind to skin and not wash off easily thereby reducing secretions from the skin). Use of zinc oxide in non-alcohol containing compositions is known, e.g., co-pending and commonly assigned U.S. patent application, Serial No. 09/205,209, entitled SKIN CARE COMPOSITIONS CONTAINING ZINC SALTS AND RETINOIDS, discloses compositions of zinc oxide and retinoids in non-alcohol containing skin care compositions such as both water-in-oil and oil-in-water emulsions.

While not wishing to be bound by any particular theory, it is believed that the metal oxides used in this invention provide a means for the hydrophilic oil to disperse into and avoid separating out particularly after an extended period of time or under accelerated aging conditions. Thus, it is believed that the metal oxides useful in this invention should be of sufficient surface area to disperse the amount of hydrophilic oil used in the compositions. Good results have been achieved with Zinc Corporation of America's USP-1 grade (0.12 micron, 9 m²/g S.A.) as well as H&R's Zinc Oxide neutral H&R (2 micron, 30-70 m²/g S.A.)to have a stabilizing effect on formulations. Both of these are considered high surface area grades (microfine Zinc Oxide) as opposed to something like Zinc Corporation of America's USP-2 (0.3 micron, 3 m²/g S.A.). It is not clear what is the minimum surface area that would be required to give the enhanced stability, however the precise surface area can be easily determined by one skilled in the art and is known to depend on such variables as the precise amount of hydrophilic oil and alcohol used in the antimicrobial composition. Furthermore, one skilled in the art would appreciate that mean particle size of the metal oxide used will also correlate to the specific surface area of the material; lower particle sizes yield high surface areas. The particle size influences the opacity of the finished product.

The alcohol used with the composition of this invention is typically present in an amount ranging from 50 to 90 (v/v%), preferably 60 to 75 (v/v%), most preferably from 60 to 70 (v/v%) of the composition. The alcohols useful in the present invention include ethyl alcohol, isopropyl alcohol, n-propyl alcohol and combinations thereof. Ethyl alcohol may be used as the only alcohol or the alcohol may be a mixture from 10 to 70% by volume ethyl alcohol, from 10 to 70% by volume isopropyl alcohol, and from 10 to 70 % by volume n-propyl alcohol.

Hydrophilic oil skin conditioners are oils that are miscible (i.e., do not form emulsions) with water, alcohol and hydrophobic oils. Examples of hydrophilic oils useful in this invention include isolene (C₁₂-C₁₈ diglycerides - Vevy Europe), polyethylene glycol derivatives of mono- and di-glycerides (e.g., Lexol ES - Inolex), silicone polyethers (such as dimethicone copolyol), organosilane quaternary ammonium compounds(e.g., Lambent Quat AD (Lambent Technologies)), and liquid fatty alcohols (e.g., oleyl alcohol). It should be noted that the presence of the fatty amido group in Lambent Quat AD provides a extra degree of flexibility in formulating alcohol-surfactant systems. These components may be present in the composition if this invention in any effective amount. Typically these effective amounts are from 0.1 to 5.0, preferably from 0.25 to 2.5, and most preferably from 0.25 to 1.5 weight percent of the composition based on each individual component.

Dispersing oils are those oils that may be used to carry (disperse) both compatible and incompatible ingredients. Examples of dispersing oils are hydrophobic oils that are compatible (miscible) with other types of hydrophobic oils but are miscible with water depending on whether the ingredient is a salt. More specific examples of dispersing oils useful in this invention include dimethicone, phenylethyl dimethicone, phosphate esters, such as cocamidopropyl phosphatidyl PG-dimonium chloride (Phospholipid PTC- Uniquema), linoleamidopropyl phosphatidyl PG-dimonium chloride (Phospholipid EFA- Uniquema), coco phosphatidyl PG-dimonium chloride (Phospholipid CDM, Uniquema), and borageamidopropyl phosphatidyl PG-dimonium chloride (Phospholipid GLA- Uniquema). These components may be present in the composition if this invention in any effective amount. Typically these effective amounts are from 0.1 to 5.0, preferably from 0.5 to 2.5, and most preferably from 1.0 to 1.5 weight percent of the composition based on individual component.

Thickeners useful in this invention include hydroxypropylcellulose, hydroxyethylcellulose, hydroxypropylmethylcellulose, PEG-4 Cellulose, xanthan gum, guar gum and derivatives thereof. These components may be present in the composition if this invention in any effective amount and mixed in any proportions. Typically these effective amounts are from 0.1 to 2.0, preferably from 0.2 to 1.5, and most preferably from 0.4 to 1.2 weight percent of the composition based on individual component.

Humectants useful in this invention include glycerin, propylene glycol, sodium salt of pyroglutamic acid (sodium PCA), lactamide MEA (monoethanolamine) and acetamide MEA (Incromectant LAMEA- Croda). These components may be present in the composition if this invention in any effective amount. Typically these effective amounts are from 0.1 to 40.0, preferably from 1.0 to 20.0, and most preferably from 2.0 to 15.0 weight percent of the composition.

Fatty acid esters useful in this invention include phosphate esters, emollient esters (such as isopropyl myristate and PEG-7 glyceryl cocoate (Cetiol HE- Henkel) and C₁₂-C₁₅ alcohols lactate (Ceraphyl 41- ISP). These components may be present in the composition if this invention in any effective amount. Typically these effective amounts are from 0.1 to 5.0, preferably from 0.25 to 2.5 , and most preferably from 0.5 to 2.0 weight percent of the composition based on individual component.

Percutaneous enhancers are compounds which enhance the absorption rate of skin conditioners and other active ingredients into, for example, the epidermis of the skin. Percutaneous enhancers useful in this invention include propylene glycol, phenoxyethanol, Sodium PCA, propylene carbonate, and polyester topical delivery systems such as Polyolprepolymer-2 (Penederm), Lexorez 100, Lexorez TC8, and Lexorez TL8 (Inolex). These components may be present in the composition if this invention in any effective amount. Typically these effective amounts are from 0.1 to 10.0, preferably from 0.5 to 5.0, and most preferably from 0.5 to 2.5 weight percent of the composition based on individual component.

Surfactants useful in this invention include non-ionic, amphoteric and cationic surfactants. These components may be present in the composition if this invention in any effective amount. Typically these effective amounts are from 1 to 20, preferably from 1 to 10, and most preferably from 1 to 5 weight percent of the composition based on individual surfactant.

Amphoteric surfactants are molecules having both positive and negative charges on the molecule. Examples of suitable amphoteric surfactants include those related or derived from betaines such as amine betaines and amido betaines. Also useful amphoteric surfactants include glycinate and/or imidazole derivatives such as coco-imidazoline mono-carboxylate and/or dicarboxylate. Preferred amphoteric surfactants for use with this invention include hydroxysultaine, cocamidopropyl betaine, sodium lauriminodipropionate, and disodium lauroamphodiacetate.

Non-ionic surfactants are neutral molecules without any charge, and these compounds are very mild with poor foaming properties. Non-ionic compounds diminish surface tension and dissolve in water quite easily, but not in same way as common salt. They are equally soluble in oil, which is important in producing emulsions. In the presence of water, they do not form simple solutions, they form complexes known as hydrates. Applications for nonionics include solubilization and for cationics, conditioning. Examples include alkyl phenol ethoxylates, fatty acid dialkanolamides, fatty acid monoalkanolamides, fatty acid ethoxylates, fatty alcohol ethoxylates, fatty amine ethoxylates, substituted phenol ethoxylates, vegetable oil ethoxylates, polyalkylglycosides, sucrose esters and glyceryl laurate.

Generally, preferred nonionic surfactants include condensation products of one or more alkylene oxide groups with an organic hydrophobic compound, such as an aliphatic or alkyl aromatic compound. Exemplary nonionic surfactants based upon polyethoxylated, polyproproxylated, or polyglyceroxylated alcohols, alkylphenols, or fatty acids.

Further specific examples of nonionic surfactants include, for example, alkyl phenoxypolyethoxy ethanols having alkyl groups from about 7 to 18 carbon atoms and from about 6 to about 60 oxyethylene units such as, for example, heptyl phenoxypolyethoxyethanols, ethylene oxide derivatives of long chained carboxylic acids such as lauric acid, myristic acid, palmitic acid, oleic acid, and the like, or mixtures of acids such as those found in tall oil containing from about 6 to 60 oxyethylene units; ethylene oxide condensates of long-chained alcohols such as octyl, decyl, lauryl, or cetyl alcohols containing from 6 to 60 oxyethylene units; ethylene oxide condensates of long-chain or branched chain amines such as dodecyl amine, hexadecyl amine, and octadecyl amine, containing from about 6 to 60 oxyetheylene units; and block copolymers of ethylene oxide sections combined with one of more hydrophobic propylene oxide sections.

Examples of cationic surfactants include, for example, lauryl pyridinium chloride, cetyldimethyl amine acetate, and alkyldimethylbenzylammonium chloride, in which the alkyl group has from 8 to 18 carbon atoms.

Other useful cationic surfactants include aliphatic fatty amines and their derivatives, homologues of aromatic amines having fatty chains - dodecylaniline, fatty amides derived from aliphatic diamines, fatty amides derived from disubstituted amines, quaternary ammonium compounds, amides derived from aminoalcohols and their quaternary ammonium derivatives, quaternary ammonium bases derived from fatty amides of disubstituted diamines, quaternary ammonium bases of the benzimidazolines, basic compounds of pyridinium and its derivatives, quaternary ammonium compound of betaine, dimethylphenylbenzyl ammonium chloride, urethanes or basic salts of ethylene diamine, polyethylene diamines and their quaternary ammonium compounds.

A particularly useful mixture of surfactants comprise from 0.1 to 10% active weight % of cocamidopropyl hydroxysultaine (amphoteric surfactant), from 0.1 to 10% active weight % of polyalkylglycoside (preferably Plantaren 2000 from Henkel)or glyceryl laurate (Henkel), nonionic surfactant, and from 0.1 to 10 by active weight % of PPG-40 diethylmonium chloride (preferably Emcol CC-42 from Witco Chem. Co.), cationic surfactant.

The mixture of amphoteric, nonionic, and cationic surfactants of this invention have been shown to be an effective surfactant system compatible with high alcohol and low water systems, thereby resulting in a stable formulation. Desirably, the surfactants system contain only non-ionic and cationic surfactants.

Antimicrobial agents useful in this invention include benzalkonium chloride, benzethonium chloride, methyl benzethonium chloride, cetylpyridinium chloride, cetrimonium chloride, cetrimonium bromide (cetrimide), Cosmocil CQ (20% polyhexamethylene biguanide (PHMB)), and chlorhexidine gluconate. These components may be present in the composition if this invention in any effective amount. Typically these effective amounts (total actives) are from 0.01 to 5.0, preferably from 0.02 to 2.0, and most preferably from 0.02 to 1.0 weight percent of the composition based on individual components.

In practicing the method of preparing the metal oxide containing compositions, it was found advantageous to first form a paste. Even though this method of incorporation of metal oxides proved useful in stabilizing a large number of antimicrobial solutions, some of the zinc oxide containing formulations actually suffered a loss in antimicrobial efficacy as what is believed to have occurred with some of the preservatives used.

Thus, in subsequent formula preparations the zinc oxide was substituted with MEARLMAID OL pigment (a pearlescent agent available for Engelhard Corporation) that has guanine, polysorbate 80 and isopropyl alcohol. Several prototypes were prepared using this compound to get the lotion look to the product. These formulations have exhibited discoloration and also separation of thickener (hydroxypropylcellulose) that was identified through a systematic approach by preparing base formula with water, hydroxypropylcellulose and alcohol. Due to these reasons the processing step of hydroxypropylcellulose has been changed to achieve a stable thick gel as described in Example 3. However, the improved process has not shown any separation but the color of the product changed to off white over period of 7 days at 40 degrees as well at 50 degrees. This discoloration was due to the amphoteric surfactant present in the formula. This was identified through preparation of series of samples with base containing hydroxypropylcellulose, alcohol, water and other ingredients.

Due to these poor stability reasons the amphoteric surfactant was dropped from the formula and prepared a series of samples with polyhexamethylene biguanide (PHMB) at three concentrations, 0.2, 0.25, and 0.3% along with other ingredients. These samples were placed at 40 and 50 degrees to establish the stability profile over 3 months time. The samples have shown excellent stability profile in appearance, pH, and viscosity, and thus formulas even without containing zinc oxide and isolene but with MEARLMAID OL have ingredients have displayed compatibility and stability as shown in Example 3.

The following trade name additives are useful in making formulations of this invention and the examples:
AMP 95 is a mixture of 2-amino-2-methyl-1-propanol, 2-(methylamino)-2-methyl-1-propanol and water in a ratio of from about 90:5:5, commercially available from Angus Chemical Company.
ACRITAMER® 505E. a polyvinyl carboxy polymer crosslinked with ethers of pentaerythritol, R.I.T.A available from Crystal Lake, IL.
AMPHOTERGE K-2, coco imidazoline dicarboxylate, available from Lonza.
ESS 9090IC is a fragrance, available from Givuan-Roure Corporation.
CERAPHYL 28 is a mixture of cetyl alcohol and cetyl lactate, a waxy solid commercially available for ISP Van Dyk Inc.
CERAPHYL 41 is a mixture of C₁₂ - C₁₅ alcohol lactates, available from ISP Van Dyk Inc.
CETIOL HE- PEG-7 glyceryl cocoate, from Henkel.
COSMOCIL CQ is polyhexamethylene biguanide, available from Zeneca.
DISODIUM EDTA, U.S.P., available from Dow Chemical as Versene NA.
DOW CORNING® 580 wax is a mixture of stearoxy trimethoxy silane and stearyl alcohol.
DOWICIL 200, quaternium 15, Dow Chemical.
EMCOL CC42- PPG-40 dimonium chloride, or quaternium 21, available from Witco Corp. (cationic surfactant)
GERMABEN II is a mixture comprised of diazolindinyl urea (about 30%); methyl paraben (about 11%); propyl paraben (about 3%) and propylene glycol (about 56%), available from Sutton Laboratories.
GERMALL PLUS is a mixture of diazolidinyl urea (about 99%), 3-Iodo-propynylbutylcarbamate available from Sutton Laboratories.
INCROMECTANT LAMEA- a mixture of acetamide monoethanolamine, and lactamide monoethanolamine (Croda)
LAMBENT QUAT AD is a silicone quaternary compound (Lambent Technologies).
LEXOREZ 100 is a saturated crosslinked hydroxy functional; polyester, comprised of glycerin, diethylene glycol, adipate crosslinked polymer, which is a viscous, hydrophobic liquid at room temperature and is dispersible in many lipids and emollients.
LEXQUAT AMG-IS, isostearamidopropyl PG dimonium chloride (Inolex Chemical Company)
MACKAM CBS-50G, cocamidopropyl hydroxysultaine, 50% (McIntyre)(amphoteric surfactant)
MEARLMAID OL contains isopropyl alcohol, guanine, and polysorbate 80 (Engelhard).
MIRATAINE CB - cocamidopropyl betaine (Rhone-Poulenc)
NATROSOL 250 HHR - hydroxyethylcellulose (Aqualon, Div. Of Hercules) .
NISIN, a 34 amino acid polypeptide, sold as Ambicin by Applied Microbiology, Inc.
ORANGE ZEST B FRAGRANCE 439.454, fragrance commercially available from Firmenich.
PEG-7 Glyceryl Cocoate (see Cetiol HE)
PEO-1 - polyethylene glycol, 21,000 M.W. INCI: PEG-5M (R.I.T.A.)
PHOSPOLIPID CDM is cocophosphatidyl (PG)-dimonium chloride, a co-synthetic, phospholipid available from Mona Industries, Inc.
PHOSPHOLIPID GLA - borageamidopropyl phosphatidyl PG-dimonium chloride (Mona).
PHOSPOLIPID PTC is cocamidopropyl phosphatidyl PG-dimonium chloride, available form Mona Industries.
PLANTAREN 2000 is decyl polyglucose, available from Henkel/Cospha. (non-ionic surfactant)
SILSOFT PEDM is phenylethyl dimethicone, available from Witco Cooperation, Osi Specialties, Inc.
SEAFOAM 143.258/GGE FRAGRANCE, available from Firmenich, Inc.
TOCOPHEROL (dl-alpha-tocopherol), Vitamin E, available from Roche Vitamins and Fine Chemicals.
TRICLOSAN - 2, 4, 4'-trichloro-2-hydoxydiphenyl ether available from Ciba Specialty Chemical Corp.)
ULTREZ® 10 a carbomer polymer, available from BF Goodrich, Cleveland Ohio, and disclosed in US patent 5, 004,598, the contents of which are incorporated by reference in its entirety.
VARISOFT 300 is a quaternary ammonium chloride, cetrimonium chloride (Witco Corp.).
   VAROX 270 lauramine oxide, 30% active of 70% C₁₂, available from Witco.
ZINC OXIDE is USP-1 grade microfine zinc oxide having approximately a 0.12 micron particle size and approximately 9 m²/g S.A. (Zinc Corp. of America).

### EXAMPLE 1

Table 1 represents three(3) formulations of varying compositions which comparatively indicate the inventive aspects of this invention relating to the inclusion of zinc oxide as stabilizing material.

**TABLE 1***

| **INGREDIENTS** | **Formulation 1-1** | **Formulation 1-2** | **Formulation 1-3** |
|---|---|---|---|
| Deionized Water | 12.74 | 26.79 | 26.84 |
| Ethanol (200 Proof) | 62.25 (70%V/V) | 47.10 (55.0 V/V%) | 47.10 (55.0 VN%) |
| Glycerin | 5.0 | 5.00 | 5.00 |
| Propylene Glycol | 5.0 | 5.00 | 5.00 |
| Plantaren 2000 | 3.6 | 3.60 | 3.60 |
| Mackam CBS-50G | 2.4 | 2.40 | 2.40 |
| Benzethonium Chloride | 0.1 | - | - |
| Benzalkonium Chloride (50%) | 0.2 | 2.00 | 2.00 |
| Phospholipid CDM | 1.5 | 1.50 | 1.50 |
| PPG-40 Diethylmonium Chloride (Emcol CC-42) | 1.2 | 1.20 | 1.20 |
| Hydroxypropylcellulose HXF Grade | 1.0 | 1.10 | 1.10 |
| Phenoxyethanol | 1.0 | 1.00 | 1.00 |
| Glyceryl Laurate | 1.00 | 1.00 | 1.00 |
| Cetrimonium Chloride (29%- Varisoft 300) | 0.86 | 0.86 | 0.86 |
| Isolene | 1.0 | 0.50 | 0.50 |
| Lambent Quat AD | 0.50 | 0.50 | 0.50 |
| Fragrance (Seafoam GGE) | -- | 0.15 | 0.15 |
| Fragrance (Orange Zest B) | 0.15 | -- | -- |
| Mearlmaid OL | 0.1 | 0.15 | |
| Cetylpyridinium Chloride | 0.1 | 0.10 | 0.10 |
| Silsoft PEDM | 0.05 | 0.05 | 0.05 |
| Dowicil 200 | 0.25 | - | - |
| Zinc Oxide (USP-1 microfine) | -- | -- | 0.10 |

| | | | |
|---|---|---|---|
| * All amounts in wt.% unless otherwise shown. | | | |

Both zero time and accelerated aged time microscopic (200X) observations were made for the foregoing formulations.

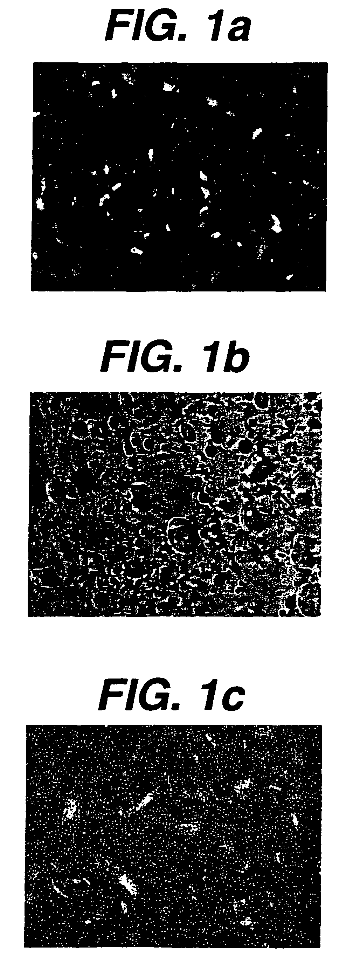

Examination of Formulation 1-1 at zero aging time and accelerated aging at 40°C for five weeks revealed that the opaque appearance of the composition was adversely affected. Visually the appearance of the aged material was less white than the appearance of unaged material. Furthermore, 200X magnification using a ZEISS, model no. Axioskop 50, microscope revealed that the aged product contained enlarged oil droplets of varying sizes (see Fig. 1(b)) whereas the unaged product contained oil droplets of more uniform size (see Fig. 1(a)). Since the opaque, lotion-like appearance of the composition is attributed to the dispersed hydrophilic oil, isolene, and the pearlescent pigment, the enlarged and varied droplets of the aged composition suggested that the aging caused an instability in the oil phase. Further aging of Formulation 1-1 for five weeks at 40°C showed a visibly separated bottom layer of isolene.

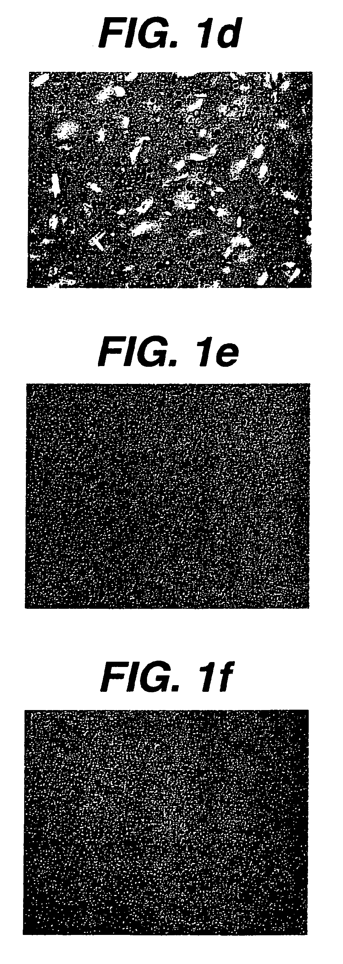

Formulation 1-2, which contained a lower level of isolene (0.5 wt.% vs. 1.0 wt.% compared with Formulation 1-1), but no zinc oxide, also proved not to provide a stable formulation. While Fig. 1(c) shows somewhat of an improvement at zero time (due to the decrease in the amount of isolene used), after accelerated aging at 50 C for 20 weeks, Fig. 1 (d) shows a marked degradation in the homogeneity of the formulation. Thus, Formulation 1-2 did not form a stable formulation.

However, Formulation 1-3 which contained isolene at 0.5 wt.% and zinc oxide at 0.1 wt.% provided a stable composition at both zero time and accelerated again at 50 °C for 20 weeks as noted by Formulation 1-2's homogeneous appearance as seen in Figs. 1(e) and 1(f).

### EXAMPLE 2

This example demonstrates a preferred method of making the compositions of this invention which contain hydrophilic oils and metal oxides. Formulation 2-1 was made according to the steps outlined below:

**TABLE 2***

| **Formulation 2-1** | **SUPPLIER** | **Wt. %** |
|---|---|---|
| DEIONIZED WATER | J&J MED. | 26.24 |
| Ethanol (200 Proof) | Aaper | 21.90 |
| n-propyl alcohol | Aldrich | 26.8 |
| Glycerin | Henkel | 5.0 |
| Propylene Glycol | Dow | 5.0 |
| Plantaren 2000 | Henkel | 3.60 |
| Mackam CBS-50G | McIntyre | 2.40 |
| Benzethonium Chloride | Lonza | 1.0 |
| Phospholipid CDM | Mona | 1.50 |
| PPG-40 Diethylmonium Chloride (Emcol CC-42) | Witco | 1.20 |
| Hydroxypropylcellulose HXF Grade | Aqualon | 1.10 |
| Phenoxyethanol | Dow | 1.00 |
| Glyceryl Laurate | Henkel | 1.00 |
| Cetrimonium Chloride (29%- Varisoft 300) | Witco | 0.86 |
| Isolene | Vevy | 0.50 |
| Lambent Quat AD | Lambent | 0.50 |
| Fragrance (Seafoam GGE) | Firmenich | 0.15 |
| Cetylpyridinium Chloride | Zeeland | 0.10 |
| Zinc Oxide (USP-1,microfine) | ZCA | 0.10 |
| Silsoft PEDM | Witco | 0.05 |

| | | |
|---|---|---|
| * All amounts in wt.% unless otherwise shown. | | |

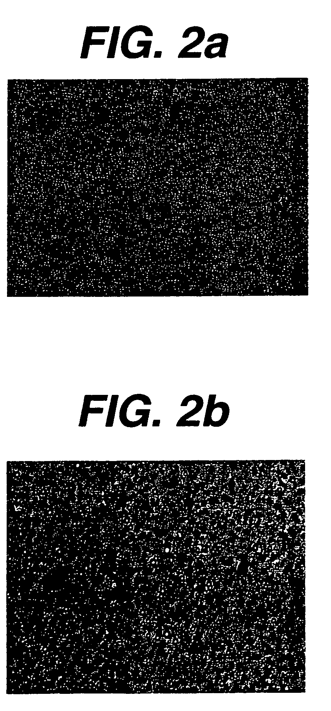

Formulation 2-1 was made according to the following steps:
1. Make a uniform paste from the glycerin and hydroxypropylcellulose.
2. Add the total amount of water to the mixture in step one at 60 C. Stir until well-distributed.
3. Make a paste of zinc oxide and one-third of the total phospholipid CDM until uniform. Add the isolene and Silsoft PEDM. Mix until a uniform paste.
4. Add the paste of step 3 into the heated mixture in step 2. Cool the mixture to room temperature, mixing to form a uniform white gel (see, Fig. 2(a)).
5. Dissolve the glyceryl laurate in the mixture of alcohols.
6. Add the alcohol-mixture of step 5 to the gel of step 4 slowly with thorough mixing, to form a uniform gel.
7. Added remaining antimicrobials, emollients, and surfactants to form the final product.

The final product of step 7 is a smooth, uniform white gel (see Fig. 2(b)).

### EXAMPLE 3

This example demonstrates a preferred method of making the compositions which do not contain any metal oxides. The formulations of Table 3 were made according to the steps outlined below:

**TABLE 3***

| **COMPONENT** | **SUPPLIER** | **Formulation 3-1** | **Formulation 3-2** |
|---|---|---|---|
| DEIONIZED WATER | J&J MED. | 15.83 | 10.08 |
| Ethanol (200 Proof) | Aaper | 62.0 | 62.5 |
| Propylene Glycol | Dow | 10.0 | 10.0 |
| Glycerin | Henkel | --- | 5.0 |
| Glyceryl Laurate | Henkel | 2.50 | 2.50 |
| Cetrimonium Chloride (29%- Varisoft 300) | Witco | 2.50 | 2.50 |
| Cosmocil CQ (20% PHMB) | Avecia | 1.25 | 1.50 |
| Phospholipid CDM | Mona | 1.50 | 1.50 |
| PPG-40 Diethylmonium Chloride (Emcol CC42) | Witco | 1.50 | 1.50 |
| Phenoxyethanol | Dow | 1.00 | 1.00 |
| Hydroxypropylcellulose HXF Grade | Aqualon | 0.80 | 0.80 |
| Lambent Quat AD | Lambent | 0.50 | 0.50 |
| Benzalkonium Chloride (50%) | Lonza | 0.20 | 0.20 |
| Fragrance (Seafoam GGE) | Firmenich | 0.15 | 0.15 |
| Benzethonium Chloride | Lonza | 0.1 | 0.1 |
| Meartmaid OL | Engelhard | 0.1 | 0.1 |
| Silsoft PEDM | Witco | 0.075 | 0.075 |

| | | | |
|---|---|---|---|
| * All amounts in wt.% unless otherwise shown. | | | |

The formulations of Table 3 was made according to the following steps:
1. Heat total amount of added deionized water to 60 C. Add the thickener (hydroxypropylcellulose) and stir to disperse thoroughly.
2. Cool mixture to room temperature, forming a thick gel.
3. With rapid stirring, add the alcohol slowly, making sure the particles of aqueous gel are thoroughly homogenized into the alcohol, forming a very clear gel.
4. The glyceryl laurate is dissolved in the alcoholic gel.
5. The antimicrobials, humectants, and conditioners are dissolved in the gel.
6. The surfactants are dissolved in the gel.
7. All remaining ingredients (opacifiers, i.e., MEARLMAID OL)are introduced with high speed mixing to form the final product.

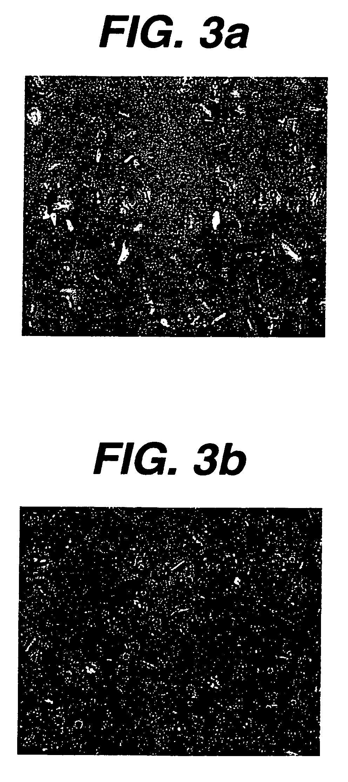

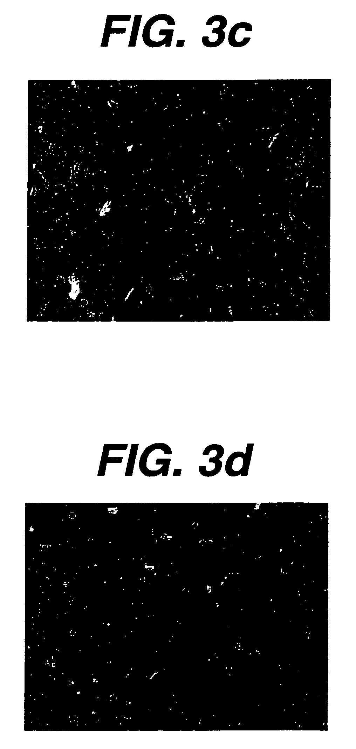

Microscopic examination of the final Formulations 3-1 and 3-2 (Figs. 3(a) and 3(c) (zero time, respectively) and Figs. 3(b) and 3(d) (accelerated aging of 50 C for 4 and 50 C for 3 weeks, respectively)) indicated stable high alcohol antimicrobial formulations were achieved. Please note that all these Figs. (i.e., Figs. 3 (a) to 3(d)), the small droplet particles are believed to be the dispersing oil of SILSOFT PEDM while the larger, fish scale-like particles are believed to be the MEARLMAID OL pigment which gives the formulation its pearlescent, lotion-like appearance. The important feature to note in these Figs. is that formulations remained stable, i.e., no noticeable changes in dispersing oil droplet size or number for the aged samples. Furthermore, none of the foregoing unaged or aged formulations showed any visible signs of phase separation and maintained a stable, unchanged color.

It should be understood that the foregoing disclosure and description of the present invention are illustrative and explanatory thereof and various changes in the size, shape and materials as well as in the description of the preferred embodiment may be made without departing from the scope of the claims.

## Claims

1. An antimicrobial composition comprising at least about 50 v/v% alcohol, an effective amount of a hydrophilic oil, an effective amount of a cationic antimicrobial compound, and an effective amount of a metal oxide.

2. The composition of claim 1, wherein the hydrophilic oil is an isolene (C₁₂₋₁₈ diglyceride), a polyethylene glycol derivative of mono or di-glyceride, a silicone polyether, a dimethicone copolyol, an organosilane quaternary ammonium compound, a liquid fatty alcohol, an oleyl alcohol or a mixture thereof.

3. The compound of claim 1 or claim 2, wherein the metal oxide is a transition or post-transition metal oxide.

4. The composition of claim 3, wherein the metal oxide is titanium dioxide, zinc oxide or a mixture thereof.

5. The composition of any one of claims 1 to 4, wherein the surface area of the metal oxide is at least 5 m²/g.

6. The composition of any one of claims 1 to 5, wherein the alcohol is ethyl alcohol, isopropyl alcohol, n-propyl alcohol or a mixture thereof.

7. The composition of any one of claims 1 to 6, wherein the cationic antimicrobial is benzalkonium chloride, methyl benzethonium chloride, benzethonium chloride, cetyl pyridinium chloride, polyhexamethylene biguanide, chlorhexidine gluconate or a mixture thereof.

8. The composition of any one of claims 1 to 7, wherein the composition further comprises an effective amount of a humectant, a phospholipid or a surfactant.

9. The composition of claim 1, wherein the alcohol comprises from 50 to 90 (v/v) percent alcohol, from 0.1 to 5.0 weight percent of a hydrophilic oil, from 0.01 to 5.0 weight percent of a cationic antimicrobial compound and from 0.01 to 1.0 weight percent of a metal oxide.

10. The composition of claim 9, comprising from 65 to 75 (v/v) percent ethanol, from 0.25 to 2.5 weight percent isolene (C₁₂-C₁₈ diglyceride), from 0.02 to 5.0 weight percent methyl benzethonium chloride, benzethonium chloride, benzalkonium chloride, cetrimonium chloride, cetylpyridium chloride, polyhexamethylene biguanide, chlorhexidine gluconate or a mixture thereof, and from 0.05 to 0.5 weight percent of zinc oxide.

11. The composition of any one of claims 1 to 10, further comprising from 0.1 to 2.0 weight percent of a thickener selected from hydroxypropylcellulose, hydroxethylcellulose, hydroxypropylmethylcellulose, PEG-4 cellulose xanthan gum, guar gum, a derivative thereof or a mixture thereof.

12. The composition of claim 11, further comprising from 0.1 to 5.0 weight percent of dispersing oil selected from dimethicone, phenylethyl dimethicone or a phosphate ester or a mixture thereof.

13. The composition of claim 12 wherein the phosphate ester is cocamidopropyl phosphatidyl PG-dimonium chloride, linoleamidopropyl phosphatidyl PG-dimonium chloride, coco phosphatidyl PG-dimonium chloride, borageamidopropyl phosphatidyl PG-dimonium chloride or a mixture thereof.

14. The composition of claim 13, further comprising from 0.1 to 40.0 weight percent of a humectant selected from glycerin, propylene glycol, a sodium salt of pyroglutamic acid (sodium PCA), lactamide MEA (monoethanolamine), acetamide MEA or a mixture thereof.

15. The composition of claim 14, further comprising from 0.1 to 5.0 weight percent of a fatty acid ester wherein the fatty acid ester is an emollient ester.

16. The composition of claim 15, wherein the emollient ester is isopropyl myristate, PEG-7 glyceryl cocoate, C₁₂-C₁₅ alcohols lactate or a mixture thereof.

17. The composition of claim 16, further comprising from 0.1 to 10.0 weight percent of a percutaneous enhancer selected from propylene glycol, phenoxyethanol, sodium PCA (pyroglutamic acid), propylene carbonate, a polyester topical delivery system or a mixture thereof.

18. The composition of claim 17, further comprising from 1.0 to 25.0 weight percent of a surfactant system comprising a non-ionic, amphoteric or cationic surfactant or a mixture thereof.

19. The composition of claim 18, wherein the nonionic surfactant is an alkyl phenol ethoxylate, fatty acid dialkanolamide, fatty acid monoalkanolamide, fatty acid ethoxylate, fatty alcohol ethoxylate, fatty amine ethoxylate, substituted phenol ethoxylate, vegetable oil ethoxylate, polyalkylglycoside, sucrose ester or glyceryl laurate; the amphoteric surfactant is an hydroxysultaine, cocamidopropyl betaine, sodium lauriminodipropionate, or disodium lauroamphodiacetate; and the cationic surfactant is lauryl pyridinium chloride, cetyldimethyl amine acetate, PPG-40 diethylmonium chloride or alkyldimethylbenzylammonium chloride; in which the alkyl group has from 8 to 18 carbon atoms.

20. The composition of claim 19 wherein the surfactant system consists of nonionic and cationic surfactants.

21. The composition of claim 20 further comprising a pearlescent pigment.

22. The composition of claim 21 wherein the pigment comprises iospropyl alcohol, guanine and polysorbate 80.

## Patentansprüche

1. Antimikrobielle Zusammensetzung umfassend wenigstens etwa 50 v/v% Alkohol, eine wirksame Menge eines hydrophilen Öls, eine wirksame Menge einer kationischen antimikrobiellen Verbindung und eine wirksame Menge eines Metalloxids.

2. Zusammensetzung nach Anspruch 1, wobei das hydrophile Öl ein Isolen (C₁₂₋₁₈₋Diglycerid), ein Polyethylenglykolderivat von Mono oder Diglycerid, ein Silikonpolyether, ein Dimethicon-Copolyol, eine Organosilan-Quartäre-AmmoniumVerbindung, ein flüssiger Fettalkohol, ein Oleylalkohol oder eine Mischung derselben ist.

3. Verbindung nach Anspruch 1 oder Anspruch 2, wobei das Metalloxid ein Übergangs- oder Nachübergangsmetalloxid ist.

4. Zusammensetzung nach Anspruch 3, wobei das Metalloxid Titandioxid, Zinkoxid oder eine Mischung derselben ist.

5. Zusammensetzung nach einem der Ansprüche 1 bis 4, wobei die Oberfläche des Metalloxids wenigstens 5 m²/g ist.

6. Zusammensetzung nach einem der Ansprüche 1 bis 5, wobei der Alkohol Ethylalkohol, Isopropylalkohol, n-Propylalkohol oder eine Mischung derselben ist.

7. Zusammensetzung nach einem der Ansprüche 1 bis 6, wobei die kationische antimikrobielle Verbindung Benzalkoniumchlorid, Methylbenzethoniumchlorid, Benzethoniumchlorid, Cetylpyridiniumchlorid, Polyhexamethylenbiguanid, Chlorhexidingluconat oder eine Mischung derselben ist.

8. Zusammensetzung nach einem der Ansprüche 1 bis 7, wobei die Zusammensetzung ferner eine wirksame Menge eines Feuchthaltemittels, eines Phospholipids oder eines Tensids umfasst.

9. Zusammensetzung nach Anspruch 1, wobei der Alkohol 50 bis 90 (v/v)-Prozent Alkohol, 0,1 bis 5,0 Gewichtsprozent eines hydrophilen Öls, 0,01 bis 5,0 Gewichtsprozent einer kationischen antimikrobiellen Verbindung und 0,01 bis 1,0 Gewichtsprozent eines Metalloxids umfasst.

10. Zusammensetzung nach Anspruch 9, umfassend 65 bis 75 (v/v)-Prozent Ethanol, 0,25 bis 2,5 Gewichtsprozent Isolen (C₁₂-C₁₈-Diglycerid), 0,02 bis 5,0 Gewichtsprozent Methylbenzethoniumchlorid, Benzethoniumchlorid, Benzalkoniumchlorid, Cetrimoniumchlorid, Cetylpyridiumchlorid, Polyhexamethylenbiguanid, Chlorhexidingluconat oder eine Mischung derselben, und 0,05 bis 0,5 Gewichtsprozent Zinkoxid.

11. Zusammensetzung nach einem der Ansprüche 1 bis 10, weiter umfassend 0,1 bis 2,0 Gewichtsprozent Verdickungsmittel ausgewählt aus Hydroxypropylcellulose, Hydroxyethylcellulose, Hydroxypropylmethylcellulose, PEG-4-Cellulosexanthan, Guargummi, ein Derivat derselben oder eine Mischung derselben.

12. Zusammensetzung nach Anspruch 11, weiter umfassend 0,1 bis 5,0 Gewichtsprozent dispergierendes Öl ausgewählt aus Dimethicon, Phenylethyldimethicon oder einem Phosphatester oder einer Mischung derselben.

13. Zusammensetzung nach Anspruch 12, wobei der Phosphatester Cocamidopropylphosphatidyl-PG-Dimoniumchlorid, Linoleamidopropylphosphatidyl-PG-Dimoniumchlorid, Cocophosphatidyl-PG-Dimoniumchlorid, Borageamidopropylphosphatidyl-PG-Dimoniumchlorid oder eine Mischung derselben ist.

14. Zusammensetzung nach Anspruch 13, weiter umfassend 0,1 bis 40,0 Gewichtsprozent eines Feuchthaltemittels ausgewählt aus Glycerin, Propylenglykol, einem Natriumsalz von Pyroglutaminsäure (Natrium-PCA), Lactamid-MEA (Monoethanolamin), Acetamid-MEA oder einer Mischung derselben.

15. Zusammensetzung nach Anspruch 14, weiter umfassend 0,1 bis 5,0 Gewichtsprozent eines Fettsäureesters, wobei der Fettsäureester ein Erweichungsmittelester ist.

16. Zusammensetzung nach Anspruch 15, wobei der Erweichungsmittelester Isopropylmyristat, PEG-7-Glyceryl-Cocoat, C₁₂-C₁₅-Alkohollactat oder eine Mischung derselben ist.

17. Zusammensetzung nach Anspruch 16, weiter umfassend 0,1 bis 10 Gewichtsprozent eines perkutanen Anreicherungsmittels ausgewählt aus Propylenglykol, Phenoxyethanol, Natrium-PCA (Pyroglutaminsäure), Propylencarbonat, einem topischen Polyesterliefersystem oder einer Mischung derselben.

18. Zusammensetzung nach Anspruch 17, weiter umfassend 1,0 bis 25,0 Gewichtsprozent eines Tensidsystems umfassend ein nicht-ionisches, amphoteres oder kationisches Tensid oder eine Mischung derselben.

19. Zusammensetzung nach Anspruch 18, wobei das nicht-ionische Tensid ein Alkylphenolethoxylat, Fettsäuredialkanolamid, Fettsäuremonoalkanolamid, Fettsäureethoxylat, Fettalkoholethoxylat, Fettaminethoxylat, substituiertes Phenolethoxylat, Ethoxylat eines pflanzlichen Öls, Polyalkylglykosid, Sucroseester oder Glyceryllaurat; das amphotere Tensid ein Hydroxysultain, Cocamidopropylbetain, Natriumlauriminodipropionat, oder Dinatriumlauroamphodiacetat; und das kationische Tensid Laurylpyridiniumchlorid, Cetyldimethylaminacetat, PPG-40-Diethylmoniumchlorid oder Alkyldimethylbenzylammoniumchlorid, wobei die Alkylgruppe 8 bis 18 Kohlenstoffatome aufweist, ist.

20. Zusammensetzung nach Anspruch 19, wobei das Tensidsystem aus nicht-ionischen und kationischen Tensiden besteht.

21. Zusammensetzung nach Anspruch 20, weiter umfassend ein Perlglanzpigment.

22. Zusammensetzung nach Anspruch 21, wobei das Pigment Isopropylalkohol, Guanin und Polysorbat 80 umfasst.

## Revendications

1. Composition antimicrobienne qui contient au moins environ 50 % en volume d'alcool, un volume efficace d'une huile hydrophile, un volume efficace d'un composé antimicrobien cationique et un volume efficace d'un oxyde de métal.

2. Composition selon la revendication 1, dans laquelle l'huile hydrophile est un isolène (diglycéride C₁₂₋₁₈), un dérivé polyéthylène glycol de mono- ou diglycéride, un polyéther de silicone, un diméthicone copolyol, un composé d'ammonium quaternaire et d'organosilane, un alcool gras liquide, un alcool oléique ou un mélange de ceux-ci.

3. Composé selon la revendication 1 ou la revendication 2, dans lequel l'oxyde de métal est un oxyde de métal de transition ou post-transitoire.

4. Composition selon la revendication 3, dans laquelle l'oxyde de métal est du dioxyde de titane, de l'oxyde de zinc ou un mélange de ceux-ci.

5. Composition selon l'une quelconque des revendications 1 à 4, dans laquelle la surface de contact de l'oxyde de métal est d'au moins 5 m²/g.

6. Composition selon l'une quelconque des revendications 1 à 5, dans laquelle l'alcool est de l'alcool éthylique, de l'alcool isopropylique, de l'alcool n-propylique ou un mélange de ceux-ci.

7. Composition selon l'une quelconque des revendications 1 à 6, dans laquelle l'antimicrobien cationique est du chlorure de benzalkonium, du chlorure de benzéthonium de méthyle, du chlorure de benzéthonium, du chlorure de cétylpyridinium, du polyhexaméthylène biguanide, du gluconate de chlorhexidine ou un mélange de ceux-ci.

8. Composition selon l'une quelconque des revendications 1 à 7, dans laquelle la composition contient en outre un volume efficace d'un humectant, d'un phospholipide ou d'un tensioactif.

9. Composition selon la revendication 1, dans laquelle l'alcool contient de 50 à 90 pour cent (en volume) d'alcool, de 0,1 à 5,0 pourcent en poids d'une huile hydrophile, de 0,01 à 5,0 pourcent en poids d'un composé antimicrobien cationique et de 0,01 à 1,0 pourcent en poids d'un oxyde de métal.

10. Composition selon la revendication 9, qui contient de 65 à 75 pourcent (en volume) d'éthanol, de 0,25 à 2,5 pourcent en poids d'isolène (diglycéride C₁₂-C₁₈), de 0,02 à 5,0 pourcent en poids de chlorure de benzéthonium de méthyle, de chlorure de benzéthonium, de chlorure de benzalkonium, de chlorure de cétrimonium, de chlorure de cétylpyridinium, de polyhexaméthylène biguanide, de gluconate de chlorhexidine ou d'un mélange de ceux-ci, et de 0,05 à 0,5 pourcent en poids d'oxyde de zinc.

11. Composition selon l'une quelconque des revendications 1 à 10, comprenant en outre de 0,1 à 2,0 pourcent en poids d'un épaississant sélectionné entre l'hydroxypropylcellulose, l'hydroxyéthylcellulose, l'hydroxypropylméthylcellulose, la cellulose PEG-4, la gomme xanthane, la gomme de guar, un dérivés ou un mélange de ceux-ci.

12. Composition selon la revendication 11, comprenant en outre de 0,1 à 5,0 pourcent en poids d'huile dispersante sélectionnée entre le diméthicone, le diméthicone phényléthylique, un ester phosphorique ou un mélange de ceux-ci.

13. Composition selon la revendication 12, dans laquelle l'ester phosphorique est du chlorure de cocamidopropyle phosphatidyle PG-dimonium, du chlorure de linoléamidopropyle phosphatidyle PG-dimonium, du chlorure de coco phosphatidyle PG dimonium, du chlorure de boragéamidopropyle phosphatidyle PG-dimonium ou un mélange de ceux-ci.

14. Composition selon la revendication 13, comprenant en outre de 0,1 à 40,0 pourcent en poids d'un humectant sélectionné entre la glycérine, le propylèneglycol, un sel de sodium d'acide pyroglutamique (sodium PCA), le lactamide MEA (monoéthanolamine), l'acétamide MEA ou un mélange de ceux-ci.

15. Composition selon la revendication 14, comprenant en outre de 0,1 à 5,0 pourcent en poids d'un ester d'acide gras dans laquelle l'ester d'acide gras est un ester émollient.

16. Composition selon la revendication 15, dans laquelle l'ester émollient est du myristate d'isopropyle, du PEG-7 cocoate de glycéryle, du lactate d'alcools C₁₂-C₁₅ ou un mélange de ceux-ci.

17. Composition selon la revendication 16, comprenant en outre de 0,1 à 10,0 pourcent en poids d'un élément facilitant le passage percutané, sélectionné entre le propylèneglycol, le phénoxyéthanol, le sodium PCA (acide pyroglutamique), le carbonate de propylène, un dispositif d'administration locale en polyester ou un mélange de ceux-ci.

18. Composition selon la revendication 17, comprenant en outre de 1,0 à 25,0 pourcent en poids d'un système tensioactif contenant un tensioactif non-ionique, amphotère ou cationique ou un mélange de ceux-ci.

19. Composition selon la revendication 18, dans laquelle le tensioactif non-ionique est un éthoxylate de phénol alkylé, un dialcanolamide d'acide gras, un monoalcanolamide d'acide gras, un éthoxylate d'acides gras, un éthoxylate d'alcools gras, un éthoxylate d'amines grasses, un éthoxylate de phénol substitué, un éthoxylate d'huile végétale, un glycoside polyalkyl, un ester de saccharose ou un laurate de glycéryle ; le surfactant amphotère est une hydroxysultaine, une bétaïne de cocamidopropyle, un lauriminodipropionate de sodium, un lauroamphodiacétate de disodium ; et le tensioactif cationique est un chlorure de laurylpyridinium, un acétate d'amine cétyldiméthyl, un chlorure de diéthylammonium PPG-40, ou un chlorure d'alkyldiméthylbenzylammonium ; dans laquelle le groupe alkyle comprend de 8 à 18 atomes de carbone.

20. Composition selon la revendication 19, dans laquelle le système tensioactif consiste en des tensioactifs non-ioniques et cationiques.

21. Composition selon la revendication 20, comprenant en outre un pigment perlé.

22. Composition selon la revendication 21, dans laquelle le pigment contient de l'alcool isopropylique, de la guanine et du polysorbate 80.
